# EUROPEAN PATENT SPECIFICATION

(11) **EP 2 445 475 B1**
(45) Date of publication and mention of the grant of the patent: **11.01.2017**
(21) Application number: 10721425.6
(22) Date of filing: 10.05.2010
(51) Int. Cl.: A61K 8/34, A61K 8/41, A61K 8/46, A61Q 5/02, A61Q 5/12, A61K 8/31

(54) **CONCENTRATED SHAMPOO**
KONZENTRIERTES SHAMPOO
SHAMPOING CONCENTRÉ

(30) Priority: 24.06.2009 EP 09163563
(43) Date of publication of application: 02.05.2012
(73) Proprietor: Unilever PLC, London EC4Y 0DY (GB); Unilever N.V., 3013 AL Rotterdam (NL)
(72) Inventor: MURRAY, Andrew, Malcolm, Wirral Merseyside CH63 3JW (GB); PHAM, Thuy-Anh, Wirral Merseyside CH633JW (GB)
(74) Representative: Chisem, Janet
(86) International application number: PCT/EP2010/056335
(87) International publication number: WO 2010/149425

(56) References cited:
- WO-A-2005/034895
- FR-A- 2 262 106
- US-A1- 2006 024 256

## Description

The present invention relates to a concentrated shampoo composition.

Shampoo compositions are disclosed in US2006/0024256, FR 2262106 and WO2005/034895.

Despite the prior art there remains a need for improved concentrated shampoo compositions which provide a conditioning benefit to the hair.

Accordingly, the present invention provides a concentrated shampoo composition according to claim 1.

We have surprisingly found that we can disperse high levels of short chain diol in a concentrated shampoo in such a manner that we can additionally entrain oil in the composition without significantly reducing the viscosity of the composition and at the same time providing a conditioning benefit. The conditioning benefit is achieved without any of the expected consumer negatives, i.e. clean hair feel is not reduced.

The short chain diol has from 3 to 7 carbon atoms and more preferably 3 or 4 carbon atoms.

More preferably, the short chain diol is selected from 1, 2 butylene glycol, 1, 3 butylene glycol, 1,4 butylene glycol, 1, 2 propylene glycol, 1, 3 propylene glycol and mixtures thereof. Especially preferably, the short chain diol is selected from 1, 3 butylene glycol and 1, 2 propylene glycol.

In the most preferred embodiment the short chain diol is 1, 2 propylene glycol. Preferably, the oil is a low viscosity oil and has a viscosity of from 1 to 500 cPs measured on a Brookfield viscometer at 30°C using spindle RV5 at 20rpm. Preferably, from 10 to 100% wt. of the oil has a viscosity of from 0.01 to 600 cPs as measured at 30°C according to ASTM D-445.

Preferably, the oil is present at from 0.05 to 10%, particularly from 0.2 to 5%, and especially from 0.5 to 3% by weight of the composition.

Suitable oil is selected from hydrocarbon oils, ester oils, polyolefin oils and triglyceride oils. Most preferably, the oil is light mineral oil.

### Anionic Surfactant

The shampoo comprises a cleansing surfactant. The cleansing surfactant comprises an anionic surfactant. The anionic surfactant has from 8 to 14 carbons, more preferably from 10 to 12 and most preferably 12 carbons. More preferably, these carbons are present in a single alkyl group.

Preferred anionic surfactants include alkali metal alkyl sulphates, more preferably the alkyl ether sulphates. Particularly preferred anionic cleansing surfactants include sodium lauryl ether sulphate.

The cleansing phase comprises from 27 to 70% by weight cleansing surfactant, preferably from 35 to 50% by weight of the composition.

The composition comprises from 27 to 70% wt. anionic surfactant. More preferably, the composition comprises from 30 to 50% anionic surfactant.

### Conditioning Gel Phase

The conditioning gel network comprises:
(a) fatty material;
(b) a gel network anionic surfactant comprising an alkyl group with from 16 to 30 carbons;
(c) cationic surfactant;
wherein the conditioning gel network has no overall charge or is anionic.

The cationic surfactant provides improved robustness of the fatty material/anionic surfactant gel network leading to improved conditioning benefit from a composition also comprising a non-cationic cleansing phase. The difference in carbon chain length between the anionic surfactant in the cleansing phase and the anionic surfactant in the conditioning gel significantly improve stability of the conditioning gel network and maintain its integrity in the shampoo composition.

Preferably, the anionic and cationic surfactants in the gel network contain within 4, preferably 2 carbons and most preferably the same number of carbons. More preferably, they comprise a single alkyl group of within 4, more preferably within 2 and most preferably are the same length. This assists in maintaining stability of the gel network.

Preferably, the carbons in the gel network cationic surfactant are present in a single alkyl group. More preferably the gel network cationic surfactant has from 16-30 carbons.

The oil can be dispersed in the conditioning gel phase prior to inclusion into the shampoo or added after.

### Gel Network Cationic Surfactant

Preferably, the conditioning gel network comprises a cationic surfactant having from 14 to 30 carbons.

Preferably, the carbons in the gel network cationic surfactant are present in a single alkyl group. More preferably the gel network cationic surfactant has from 16-30 carbons.

Preferably, the cationic surfactants have the formula N⁺(R¹)(R²)(R³)(R⁴), wherein R¹, R², R³ and R⁴ are independently (C₁₆ to C₃₀) alkyl or benzyl.

Preferably, one, two or three of R¹, R², R³ and R⁴ are independently (C₁₆ to C₃₀) alkyl and the other R¹, R², R³ and R⁴ group or groups are (C₁-C₆) alkyl or benzyl.

Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, stearyldimethylbenzylammonium chloride, cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention, either alone or in admixture with one or more other cationic conditioning surfactants, is a combination of (i) and (ii) below:
(i) an amidoamine corresponding to the general formula (I): 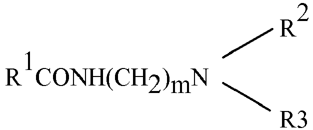 in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms, R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachid-amidoethyldiethylamine, arachidamidoethyldimethylamine, and mixtures thereof.

Particularly preferred amidoamines useful herein are
stearamidopropyldimethylamine, stearamidoethyldiethylamine, and mixtures thereof.

Commercially available amidoamines useful herein include:
stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) *in situ* in the hair treatment composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.

The level of cationic surfactant will generally range from 0.01 to 10%, more preferably 0.02 to 7.5%, most preferably 0.05 to 5% by total weight of cationic surfactant based on the total weight of the composition.

### Gel Network Fatty Material

The conditioning gel network of the compositions of the invention comprises a fatty material.

The fatty material is selected from fatty acids, fatty amides, fatty alcohols, fatty esters and mixtures thereof.

The fatty material comprises a fatty group having from 14 to 30 carbon atoms, more preferably 16 to 22. Examples of suitable fatty alcohols include cetyl alcohol, stearyl alcohol and mixtures thereof. An example of a suitable fatty ester is glyceryl monostearate.

The level of fatty material in compositions of the invention is conveniently from 0.01 to 10%, preferably from 0.1 to 5% by weight of the composition.

Preferably the ratio between fatty material and gel network anionic surfactant is from 0.1:1 to 100:1, preferably from 1.2:1 to 50:1, more preferably from 1.5:1 to 10:1 and most preferably around 2:1.

Preferably, the anionic and fatty materials of the gel network contain alkyl groups with within 4, preferably 2 carbons and most preferably the same number of carbons. More preferably, they comprise a single alkyl group of within 4, more preferably within 2 and most preferably are the same length. This assists in maintaining stability of the gel network.

### Gel Network Anionic Surfactant

The conditioning gel phase of the compositions of the invention comprise a gel network anionic surfactant

The anionic surfactant comprises an alkyl chain with from 16-30 carbons, preferably from 16-22 carbons.

Preferably, the carbons in the gel network anionic surfactant are present in a single alkyl group.

The gel network comprises an anionic surfactant for achieving an overall anionic charge to the gel network or no overall charge to the gel network.

The gel network anionic surfactant is present at from 0.1 to 5 % by weight of the composition and more preferably from 0.5 to 2.0% wt.

### Cationic Deposition Polymer

In a preferred embodiment the composition according to the invention comprises a cationic deposition polymer.

Suitable cationic deposition aid polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15 and JAGUAR C17.

Mixtures of any of the above cationic polymers may be used.

Cationic polymer will generally be present in a shampoo composition of the invention at levels of from 0.01 to 5%, preferably from 0.05 to 2%, more preferably from 0.07 to 1.2% by total weight of cationic polymer based on the total weight of the composition.

Preferably, the hair care compositions of the invention are aqueous, i.e. they have water or an aqueous solution or a lyotropic liquid crystalline phase as their major component.

Suitably, the composition will comprise from 10 to 98%, preferably from 30 to 95% water by weight based on the total weight of the composition.

### Silicone

The composition according to the invention preferably comprises a silicone.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. DC7051 is a preferred silicone. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula:

HO(CH₂CH₂O)ₓ(CH(CH₃)CH₂O)_{y}(CH₂CH₂O)ₓ H

wherein the mean value of x is 4 or more and the mean value of y is 25 or more.

Another preferred form of the surface active block copolymer is according to the following formula:

(HO(CH₂CH₂O)ₐ(CH(CH₃)CH₂O)_{b})₂-N-CH₂-CH₂-N((OCH₂CH(CH₃))_{b}(OCH₂CH₂)ₐ OH)₂

wherein the mean value of a is 2 or more and the mean value of b is 6 or more.

Mixtures of any of the above described silicone emulsions may also be used.

The above described silicone emulsions will generally be present in a composition of the invention at levels of from 0.05 to 15%, preferably from 0.5 to 12% by total weight of silicone based on the total weight of the composition.

The silicone is preferably present at from 0.5 to 15% wt., more preferably 1 to 12% by weight.

Optionally, a composition of the invention may contain further ingredients as described below to enhance performance and/or consumer acceptability.

The composition can include co-surfactants, to help impart aesthetic, physical or cleansing properties to the composition.

An example of a co-surfactant is a nonionic surfactant, which can be included in an amount ranging from 0.5 to 10%, preferably from 0.7 to 6% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco monoisopropanolamide. A particularly preferred nonionic surfactant is coco monoethanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 10%, preferably from 1 to 6% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5% by weight of the total composition.

The invention will be further illustrated by the following, non-limiting Example, in which all percentages quoted are by weight based on total weight unless otherwise stated.

### EXAMPLE 1

| **INCI name** | %ad | **Comparative 1 (Regular Shampoo)** | **Comparative 2 (Concentrate+Gel+ MO)** | **Example 1 (Concentrate+Ge I+MO+PG)** | **Example 2** | **Example 3** | **Example 4** |
|---|---|---|---|---|---|---|---|
| Sodium Laureth Sulphate | 70 | 20.00 | 34.28 | 34.28 | 34.28 | 34.28 | 34.28 |
| Cocoamidopr opyl Betaine | 30 | 5.33 | 10.66 | 10.66 | - | 10.66 | - |
| Cocamide MEA | 85 | - | - | - | 2.0 | - | 2.0 |
| Carbomer | 100 | 0.4 | - | - | - | - | - |
| Glycol Distearate | 35 | 4.0 | 4.0 | 4.0 | 4.0 | 4.0 | 4.0 |
| Dimethiconol / TEA-DOBS | 50 | 6.0 | 12.0 | 12.0 | 12.0 | 12.0 | 12.0 |
| Sodium Cetearyl sulphate | 100 | - | 0.6 | 0.6 | 0.6 | 0.6 | 0.6 |
| Cetostearyl Alcohol | 100 | - | 1 | 1 | 1 | 1 | 1 |
| Behentrimoni um Chloride | 77.5 | - | 0.06 | 0.06 | 0.06 | - | - |
| Cetyl trimethylamm onium chloride | 29.0 | - | - | - | - | 0.17 | 0.17 |
| Mineral oil | 100 | - | 1.0 | 1.0 | 1.0 | 1.0 | 1.0 |
| Parfum | 100 | 0.8 | 0.8 | 0.8 | 0.8 | 0.8 | 0.8 |
| Guar Hydroxyprop yl Trimonium Chloride | 100 | 0.15 | 0.4 | 0.4 | 0.4 | 0.4 | 0.4 |
| Propylene Glycol | 99 | - | - | 15.0 | 15.0 | 15.0 | 15.0 |
| Sodium chloride | 100 | 0.5 | - | - | - | - | - |
| DMDM Hydantoin and 3-iodo-2propynylbut yl carbamate | 50 | 0.2 | 0.2 | 0.2 | 0.2 | 0.2 | 0.2 |
| Aqua | | q.s.to 100 | q.s.to 100 | q.s.to 100 | q.s.to 100 | q.s.to 100 | q.s.to 100 |

Salon Half Head, N=36 panellists

| BENEFIT | Comparative1 (Full dose) | Comparative2 (Half Dose) |
|---|---|---|
| Moisturised Feel | 15 | 21 |
| Clean feel Panellist | 17 | 19 |

| BENEFIT | Comparative1 (Full dose) | Example1 (Half Dose) |
|---|---|---|
| Moisturised Feel | 13 | 23 (90% sign diff vs Comparative 1) |
| Clean feel Panellist | 15 | 21 |

### Process

At least 8% of water was heated to about 80°C in a side pot. To this, was added the cationic (Behenyl Trimethyl Ammonium Chloride) surfactant, fatty alcohol, secondary anionic (Sodium Cetylstearyl Sulphate) surfactant using high speed stirring. When uniform dispersion obtained, this mixture was cooled down to about 45°C with the same speed stirring. This mixture was then added to concentrated primary surfactant (Sodium Laureth Sulphate) in a high speed dispersion mixer with dual homogeniser discs and scraper. The components were mixed with high shear and under vacuum. The remaining ingredients followed under the same conditions. The oil is added after adding silicone oil. Propylene Glycol is added before adding remaining water.

## Claims

1. Concentrated shampoo composition comprising from 27 to 70% wt. cleansing surfactant which comprises anionic surfactant in an amount from 27 to 70% wt. of the total composition, a conditioning gel phase, a short chain diol having from 3 to 7 carbons and an oil, wherein the conditioning gel phase comprises:
(a) fatty material;
(b) a gel network anionic surfactant comprising an alkyl group with from 16 to 30 carbons;
(c) cationic surfactant;
wherein the conditioning gel network has no overall charge or is anionic, and wherein the conditioning gel phase fatty material has from 14 to 30 carbons and is selected from fatty alcohols, fatty acids, fatty amides and fatty esters and wherein the conditioning gel phase is made prior to addition to the cleansing surfactant.

2. Concentrated shampoo according to any preceding claim wherein the oil is selected from hydrocarbon oils, ester oils, polyolefin oils and triglyceride oils.

3. Concentrated shampoo composition according to any preceding claim wherein the short chain diol is propylene glycol,

## Patentansprüche

1. Konzentrierte Shampoozusammensetzung, die 27 bis 70 Gew.-% Reinigungstensid umfasst, das anionisches Tensid in einer Menge von 27 bis 70 Gew.-% der Gesamtzusammensetzung, eine Konditionierungsgelphase, ein kurzkettiges Diol mit 3 bis 7 Kohlenstoffatomen und ein Öl umfasst, wobei die Konditionierungsgelphase umfasst:
(a) Fettmaterial,
(b) ein anionisches Gelnetzwerk-Tensid, das eine Alkylgruppe mit 16 bis 30 Kohlenstoffatomen umfasst,
(c) kationisches Tensid,
wobei das Konditionierungsgelnetzwerk keine Gesamtladung aufweist oder anionisch ist und wobei das Konditionierungsgelphasen-Fettmaterial 14 bis 30 Kohlenstoffatome aufweist und unter Fettalkoholen, Fettsäuren, Fettamiden und Fettestern ausgewählt ist und wobei die Konditionierungsgelphase vor der Zugabe zu dem Reinigungstensid gebildet worden ist.

2. Konzentriertes Shampoo nach irgendeinem vorhergehenden Anspruch, wobei das Öl unter Kohlenwasserstoffölen, Esterölen, Polyolefinölen und Triglyceridölen ausgewählt ist.

3. Konzentrierte Shampoozusammensetzung nach irgendeinem vorhergehenden Anspruch, wobei das kurzkettige Diol Propylenglycol ist.

## Revendications

1. Composition de shampooing concentré comprenant de 27 à 70 % en poids d'un tensioactif nettoyant qui comprend un tensioactif anionique en une quantité de 27 à 70 % en poids de la composition totale, une phase de gel conditionneur, un diol à chaîne courte ayant de 3 à 7 carbones et une huile, dans laquelle la phase de gel conditionneur comprend :
(a) une matière grasse ;
(b) un tensioactif anionique en réseau de gel comprenant un groupe alkyle ayant de 16 à 30 carbones ;
(c) un tensioactif anionique ;
dans laquelle le réseau de gel conditionneur n'a pas de charge globale ou est anionique, et dans laquelle la matière grasse de la phase de gel conditionneur a de 14 à 30 carbones et est choisie parmi les alcools gras, les acides gras, les amides gras et les esters gras, et dans laquelle la phase de gel conditionneur est préparée avant addition au tensioactif nettoyant.

2. Shampooing concentré selon la revendication précédente, dans lequel l'huile est choisie parmi les huiles hydrocarbonées, les huiles d'esters, les huiles de polyoléfines et les huiles de triglycérides.

3. Composition de shampooing concentré selon l'une quelconque des revendications précédentes, dans laquelle le diol à chaîne courte est le propylèneglycol.
